Europäisches Patentamt

European Patent Office

Office européen des brevets

(19)

(11) **EP 0 619 951 B1**

(12) **EUROPEAN PATENT SPECIFICATION**

(45) Date of publication and mention
of the grant of the patent:
**02.10.2002 Bulletin 2002/40**

(51) Int Cl.[7]: **A23L 1/09**, A23L 1/236

(21) Application number: **94301892.9**

(22) Date of filing: **16.03.1994**

(54) **Use of alpha-alpha-trehalose as energy supplying source**

Verwendung von Alpha-alpha-trehalose als Energiequelle

Utilisation d' alpha-alpha-tréhalose comme source d'énergie

(84) Designated Contracting States:
**AT BE CH DE DK ES FR GB IT LI MC NL PT SE**

(30) Priority: **16.03.1993 JP 9351393**

(43) Date of publication of application:
**19.10.1994 Bulletin 1994/42**

(73) Proprietor: **KABUSHIKI KAISHA HAYASHIBARA
SEIBUTSU KAGAKU KENKYUJO
Okayama-shi Okayama (JP)**

(72) Inventors:
• **Shibuya, Takashi
Okayama (JP)**
• **Sugimoto, Toshiyuki
Okayama (JP)**
• **Miyake, Toshio
Okayama (JP)**

(74) Representative: **Daniels, Jeffrey Nicholas et al
Page White & Farrer
54 Doughty Street
London WC1N 2LS (GB)**

(56) References cited:
**EP-A- 0 462 693**     **EP-A- 0 486 315**
**EP-A- 0 532 807**     **EP-A- 0 558 213**
**EP-A- 0 606 753**     **FR-A- 2 664 472**

• **DATABASE WPI Section Ch, Week 8846 Derwent
Publications Ltd., London, GB; Class D13, AN
88-326744 & JP-A-63 240 758 (NORINSHO ET
AL.) , 6 October 1988**
• **DATABASE WPI Section Ch, Week 8846 Derwent
Publications Ltd., London, GB; Class D13, AN
88-326743 & JP-A-63 240 757 (NORINSHO ET
AL.) , 6 October 1988**
• **DATABASE WPI Section Ch, Week 8842 Derwent
Publications Ltd., London, GB; Class D17, AN
88-296359 & JP-A-63 216 492 (NORINSHO) , 8
September 1988**
• **CD Römpp Chemie Lexikon - Version 1.0,
Stuttgart/New-York: Georg Thieme Verlag 1995**
• **Dictionary of Scientific and Technical Terms,
McGraw-Hill, page 785**
• **Journal of Japanese Society and Nutrition, Vol.
25, No. 8, 1972, pages 641-646**
• **English transaltion of "Jounal of Japanese
Society Food and Nutrition", Vol. 25, No 8, 1972,
page 641 , right column, lines 6-11**
• **Iyaku Journal, vol. 12, 1973, page 1-7**
• **English translation of "Iyaku Journal", vol. 12,
1973, page 2, right column, lines 18-26**

**Description**

The present invention relates to the use of α-α-trehalose preparable by the method containing a step of allowing a non-reducing saccharide-forming enzyme to act on a partial starch hydrolysate exhibiting a reducing power to prepare a composition to be administered parenterally to living bodies for supplying energy to a living body.

Saccharides, exhibiting a reducing power, such as glucose and fructose have been used for a long time as an energy-supplementing saccharide source. These saccharides, however, have a relatively-poor storage stability because of their reducing power, and, generally they become more unstable in the coexistence of other nutritives such as amino acids and vitamins.

Therefore, establishment of an energy-supplementing saccharide source essentially consisting of a non-reducing saccharide such as xylitol, sorbitol, maltitol, lactitol, sucrose or trehalose has been in great demand. Among these non-reducing saccharides, sugar alcohols of monosaccharides such as xylitol and sorbitol have a demerit of that they may cause a serious diarrhea when administered to the body with an erroneous dose and route. Sugar alcohols of disaccharides such as maltitol and lactitol, as disclosed in Japanese Patent Publication Nos. 13,699/72 and 42,506/72, are not readily metabolized and utilized by living bodies, and actually have been used as a sweetener for diet. Thus, these disaccharides are not satisfactory as an energy-supplementing saccharide source. Sucrose has a demerit of that it is readily hydrolyzed into reducing glucose and fructose under acid conditions, followed by resulting in exhibition of a poor storage stability. As evident from the descriptions of "Trehalose is low caloric because it is not substantially assimilated and utilized by the body." and "Trehalose is not substantially hydrolyzed by amylases, etc.", as disclosed in Japanese Patent Laid-Open No.240,758/88, trehalose has been recognized as a saccharide source which does not act as an energy source *in vivo*.

EP publication no. 0486315A3 discloses a process for preparing neotrehalose and its uses, where the process comprises allowing β-galactosidase to act on a solution containing lactoneotrehalose to form neotrehalose and recovering the resultant neotrehalose. The neotrehalose is a non-reducing oligosaccharide having a satisfiable stability and a rich and high quality sweetness and is assimilated and utilised as an energy source *in vivo* when orally administered.

EP publication no. 0532807A1 discloses a composition for supplementing energy to a living body which contains neotrehalose as effective ingredient.

It has been in great demand to establish an energy-supplementing saccharide source which is readily prepared in an industrial scale and does not substantially exhibit a reducing power but has a satisfiable storage stability and an extensive applicability, and to an energy-supplement composition containing such an energy-supplementing saccharide source as an effective ingredient.

The present inventors have studied energy-supplementing saccharide sources which are readily prepared in an industrial scale. More particularly, we eagerly studied a non-reducing and stable disaccharide, i.e. trehalose, as well as its related substances. As a result, we found that trehalose (O-α-D-glucopyranosyl α-D-glucopyranoside or α, α-trehalose), prepared by a novel biochemical technique as disclosed in Japanese Patent Application Nos.362,131/92 and 265,416/93, both of which were applied by the present inventors, is readily metabolized *in vivo* and utilized by living bodies as an energy source. We also found that such a biochemical technique is a method which facilitates an industrial-scale preparation of trehalose, said method containing a step of allowing a non-reducing saccharide-forming enzyme to act on a partial starch hydrolysate exhibiting a reducing power (the wording "a partial starch hydrolysate exhibiting a reducing power" is designated as "a reducing partial starch hydrolysate" hereinafter). We further found that the trehalose prepared by the biochemical technique is an absolutely novel energy-supplementing saccharide source with an extensive applicability because of its non-reducing property, and established an energy-supplementing composition containing said trehalose as an effective ingredient. The energy-supplementing composition according to the present invention can be arbitrary used in combination with other nutritives and/or medicaments, and formed into multiple nutritive compositions and pharmaceutical compositions with an improved therapeutic efficacy because trehalose incorporated therein has a satisfactorily-high storage stability and does not substantially exhibit a reducing power.

EP publication no. 0462693A1 discloses a starch jelly candy made from a starch jelly formulation of a starch composition of first starch from a plant having a dull horny (duh) homozygous genotype and a second starch obtained from a plant having a homozygous genotype selected from the group consisting of amylose extender dull (aedu), amylose extender sugary-2 (aesu2), amylose extender dull shrunken (aedushi), dull sugary-2 (dusu2), and mixtures thereof; a sweetener; and water.

French patent publication no. 2664472A1 discloses a honey powder comprising a mixture of 5 to 45 wt% of honey and 95 to 55% of a water soluble, non-hygroscopic, sweet carrier, free from fats and proteins.

An abstract of JP 63240758, as published by Derwent publications limited database WPI Section Ch week 8846 discloses a food material containing neotrehalose (preferably more than 5 w%), and preferably sugar (e.g. saccarose glucose isomaltose isomaltotriose etc.) and their reduced forms or other natural sweeteners.

An abstract of JP-A-63240757, as published by Derwent publications limited database WPI section Ch 8846 discloses a food material containing neotrehalose and centose (esp.above 5 w%), and preferably sugars (ex.saccharose, glucose, isomaltose isomaltotriose. etc.) and their reduced form or other natural sweetening.

An abstract of JP-A-63216492 as published by Derwent publications limited database WPI section Ch week 8842 discloses a process for the preparation of a sugar mixture containing neotrehalose and centose, in which the substrate selected from starch and its decomposition product are treated with cyclodextrin-synthetase.

European Publication No. 0532807 discloses a composition for supplementing energy to a living body which contains neotrehalose as an effective ingredient. European Patent Publication No. 0532807 was published on 24th March 1993 and thus is citeable only against the present application under Article 54(3) EPC.

European Publication No. 0606753 discloses a novel non-reducing saccharide forming enzyme. The enzyme is capable of forming non-reducing saccharides having a trehalose structure when allowed to act on reducing partial starch hydrolysates. European Publication No. 0606753 was published on 20th July 1994 and thus is citeable against the present application under Article 54(3) only.

European Publication No. 0558213 discloses a novel preparation of neotrehalose. European Publication No. 0558213 was published on 1st September 1993 and thus is citeable against the present application under Article 54 (3) only.

The present invention will now be described in further detail by way of example only, with reference to the accompanying drawings, in which:

FIG.1 shows the time course of blood-sugar level of human administered orally with trehalose or glucose.
FIG.2 shows the time course of insulin level of human administered orally with trehalose or glucose.
FIG.3 shows the time course of blood-sugar level of rabbit received with a rapid parenteral administration of trehalose or glucose.
FIG.4 shows the time course of insulin level of rabbit received with a rapid parenteral administration of trehalose or glucose.
FIG.5 shows the time course of blood-sugar level of rabbit received with a slow parenteral administration of trehalose or glucose.
FIG.6 shows the time course of insulin level of rabbit received with a slow parenteral administration of trehalose or glucose.
In all the figures, the solid lines mean the dynamics of trehalose, and the dashed lines mean those of glucose.

The present invention relates to an energy-supplementing saccharide source and its uses, more particularly, to an energy-supplementing saccharide source essentially consisting of trehalose prepared by the method containing a step of allowing a non-reducing saccharide-forming enzyme to act on a partial starch hydrolysate exhibiting a reducing power, and to an energy-supplementing composition containing said trehalose as an effective ingredient.

The present energy-supplementing saccharide source essentially consisting of trehalose prepared by the method containing a step of allowing a non-reducing saccharide-forming enzyme to act on a reducing partial starch hydrolysate, and the present energy-supplementing composition containing said trehalose as an effective ingredient have never been reported.

The energy-supplementing saccharide source according to the present invention includes those which contain trehalose to the highest possible level, and, generally those in the form of syrup and powder with a trehalose content of 50 w/w % or higher, on a dry solid basis (the wordings "w/w %" and "on a dry solid basis" are respectively abbreviated as "%" and "d.s.b." in the specification, if not specified otherwise), preferably, those in the form of syrup and crystalline powder with a trehalose content of 80% or higher, d.s.b., more preferably, those in the form of crystalline powder and crystal with a trehalose content of 90% or higher, d.s.b.

Any preparation of trehalose can be used in the invention as long as it contains a step of allowing a non-reducing saccharide-forming enzyme to act on a reducing partial starch hydrolysate, for example, preparations, as disclosed in Japanese Patent Application Nos.362,131/92 and 265,416/93, wherein trehalose is prepared by allowing a non-reducing saccharide-forming enzyme to act on a reducing partial starch hydrolysate having a glucose polymerization degree of 3 or higher, are suitably and advantageously employed in the invention because they attain an industrial-scale preparation of trehalose.

The saccharide solutions prepared by the aforementioned preparations usually contain about 20-80% trehalose, d.s.b., together with reducing saccharides such as glucose, maltose and maltotriose, etc.

The saccharide solutions were in usual manner prepared into syrupy products by removing impurities in the solutions on gel filtration and centrifugation, purifying the resultant solutions by successive decoloration with activated charcoal and desalting with ion exchangers in H- and OH-form, and concentrating the resultant purified solutions. The syrupy products can be dried into powdery products. If necessary, products containing trehalose with the highest possible purity can be readily prepared by two or more combination use of purifications, for example, column chromatographic fractionations such as ion-exchange column chromatography and column chromatography using activated charcoal or silica gel; fractionation using organic solvents such as alcohols or acetone; separation using membranes having an adequate separability; and other methods to decompose and remove the remaining reducing saccharides by alkali-treatment or fermentation using yeasts.

More particularly, ion-exchange column chromatography is suitably used in the present invention as an industrial-scale preparation of trehalose with the highest possible purity, for example, the trehalose content in material trehalose solutions can be readily improved by removing impurities in the solutions on column chromatography using strong acidic cation exchange resins as disclosed in Japanese Patent Laid-Open Nos.23,799/83 and 72,598/83. As the column chromatography, those of fixed bed-, moving bed- and pseudomoving-methods can be arbitrarily used.

Any energy-supplementing composition can be used in the present invention as long as it contains as an effective ingredient trehalose and can supplement energy to living bodies, said trehalose being prepared by a method containing a step of allowing a non-reducing saccharide-forming enzyme to act on a reducing partial starch hydrolysate. In general, in order to attain a more satisfiable effect, trehalose is incorporated in the composition in an amount of 10% or higher, preferably, 20% or higher, d.s.b.

The present energy-supplementing composition includes those which contain trehalose alone or in combination with one or more edible substances and other substances administrable to living bodies, for example, medicaments such as proteins, amino acids, lipids, saccharides excluding trehalose, vitamins, minerals, antiseptics, enzymes, hormones and cytokines. If necessary, one or more other appropriate substances such as a taste-imparting agent, coloring agent, flavor-imparting agent, stabilizer, filler and excipient can be arbitrarily incorporated. The compositions thus obtained can be formed into an appropriate form.

The compositions are parenterally administered to living bodies and favorably metabolized and utilized *in vivo* as an energy-supplementing substance without a fear of causing toxicity and side effects.

The dose of the present energy-supplementing saccharide source can be appropriately chosen from a dose in the range of about 1-1,000 g/day/adult, preferably, in the range of about 5-500 g/day/adult of trehalose, d.s.b.

The present energy-supplementing saccharide source and composition containing the same can be favorably administered to human, domestic animals such as cow and horse, and pet animals such as dog and cat, etc.

The following experiments explain the present invention in detail:

Experiment 1

Preparation of trehalose

Experiment 1-1

Preparation of non-reducing saccharide-forming enzyme

In accordance with the method as disclosed in Japanese Patent Application No.362,131/92, a seed culture of *Rhizobium* sp. M-11 (FERM BP-4130) was inoculated in a nutrient culture medium containing carbon sources, nitrogen sources and minerals, and incubated at 27°C for 36 hours under aeration-agitation conditions. After completion of the culture, the resultant culture was subjected to membrane filtration using an SF-membrane to remove cells to obtain an about 18 L filtrate which was then concentrated with a UF-membrane to obtain about one L of a solution having an activity of 17.7 units/ml of a non-reducing saccharide-forming enzyme.

Experiment 1-2

Preparation of hydrous crystalline trehalose

Forty parts by weight of "PINE-DEX #4", a reducing partial starch hydrolysate commercialized by Matsutani Chemical Ind., Co., Ltd., Kyoto, Japan, was dissolved by heating in 60 parts by weight of water, and the resultant solution was heated to 45°C, adjusted to pH 6.5, mixed with one unit/g reducing partial starch hydrolysate of a non-reducing saccharide-forming enzyme prepared by the method in Experiment 1-1, and incubated for 96 hours, followed by heating the resultant mixture at 100°C for 10 min to inactivate the remaining enzyme. The solution thus obtained was diluted to give a concentration of about 20%, d.s.b., mixed with 10 units/g reducing partial starch hydrolysate of "GLUCOZYME", a glucoamylase specimen commercialized by Nagase Biochemicals, Ltd., Kyoto, Japan, and incubated for 40 hours, followed by heating the resultant mixture to inactivate the remaining enzyme. The solution thus obtained was in usual manner decolored with activated charcoal, desalted with ion-exchange resins, and concentrated into an about 60% solution containing 29.5% trehalose, d.s.b. The concentrated solution was fed to a jacketed-stainless steel column packed with "CG 6000, Na-form", a strong acidic cation exchange resin commercialized by Japan Organo, Co., Ltd., Tokyo, Japan, and fractionated at 60°C and SV (space velocity) 0.4, followed by recovering a high trehalose content fraction containing about 90% trehalose, d.s.b. The fraction was concentrated into an about 75% solution, d.s.b., which was then transferred to a crystallizer, mixed with an about 2% hydrous crystalline trehalose as a seed crystal, heated to 50°C, gradually cooled to 25°C under gentle stirring conditions, and separated by a basket-type centrifuge. The resultant crystals were washed by spraying with a small amount of water to obtain a high-purity hydrous crystalline trehalose with a purity of 99% or higher, d.s.b.

Experiment 1-3

Preparation of crystalline trehalose powder

A massecuite with a crystallinity of about 45% was prepared by the method in Experiment 1-2, and sprayed from a nozzle equipped on the top of a drying tower at a pressure of 150kg/cm$^2$. In the spraying step, the contents were blown with 85°C hot air from the upper part of the drying tower, and the resultant crystalline powder was collected on a wire netting conveyer provided in the basement of the drying tower, and recovered while blowing with 45°C hot air sending from through under the wire netting conveyer and gradually conveying from the drying tower. The resultant crystalline powder was injected to an ageing tower and aged for 10 hours while blowing with hot air to complete the crystallization and drying, followed by recovering the resultant crystalline trehalose powder.

Example 1-4

Preparation of anhydrous crystalline trehalose powder

The syrup containing trehalose, obtained in the process of Experiment 1-2, was prepared into an about 60% solution which was then subjected to column chromatography using a strong acidic cation exchange resin in accordance with the method in Experiment 1-2 to recover a high trehalose content fraction containing about 95% trehalose, d.s.b. In accordance with the method in Japanese Patent Application No.265,416/93, the fraction was placed in a vessel and boiled up *in vacuo* into a syrup with a moisture content of about 4.0% which was then transferred to a crystallizer, mixed with one % anhydrous crystalline trehalose as a seed crystal to the syrup, d.s.b., followed by crystallizing trehalose at 95°C for 5 min under stirring conditions. The resultant was transferred to an aluminum container and aged at 100°C for 6 hours to form block which was then pulverized by a cutter and dried by a fluidized-bed dryer to obtain an anhydrous crystalline trehalose powder with a moisture content of about 0.3%.

Experiment 2

Digestion test in vitro

By using a trehalose solution of a high-purity hydrous crystalline trehalose prepared by the method in Experiment 1-2, a digestion test of trehalose was carried out *in vitro* in accordance with the method as reported by K. Okada et al. in *JOURNAL OF JAPANESE SOCIETY OF NUTRITION AND FOOD SCIENCE,* Vol.43, No.1, pp.23-29 (1990). The digestibility of trehalose was evaluated based on the decomposition rate (%) calculated by the following equation:

$$\text{Decomposition rate (\%)} = \frac{\text{Reducing sugar}}{\text{Total sugar}} \times 100$$

The results were tabulated in Table 1.

Table 1

| Enzyme | Decomposition rate (%) |
|---|---|
| Salivary amylase | 0.0 |
| Gastric acid | 0.0 |
| Pancreatic amylase | 0.0 |
| Enzyme derived from rat small intestinal mucous membrane | 1.3 |

As evident from the results in Table 1, trehalose was not substantially hydrolyzed by the enzyme derived from rat small intestinal mucous membrane. By using the rat enzyme, disaccharides were tested for their digestibility similarly as above.

The results were tabulated in Table 2.

Table 2

| Disaccharide | Decomposition rate (%) |
|---|---|
| Maltose | 80.1 |
| Sucrose | 25.1 |
| Isomaltose | 13.2 |
| Lactose | 9.7 |
| Cellobiose | 1.2 |
| Trehalose | 1.3 |

As evident from the results in Table 2, trehalose was by far less hydrolyzed by the rat enzyme than maltose.

Experiment 3

Experiment 3 is not an embodiment of the present invention, but a background example usefull for understanding the invention.

Utilization test *in vivo* by oral administration

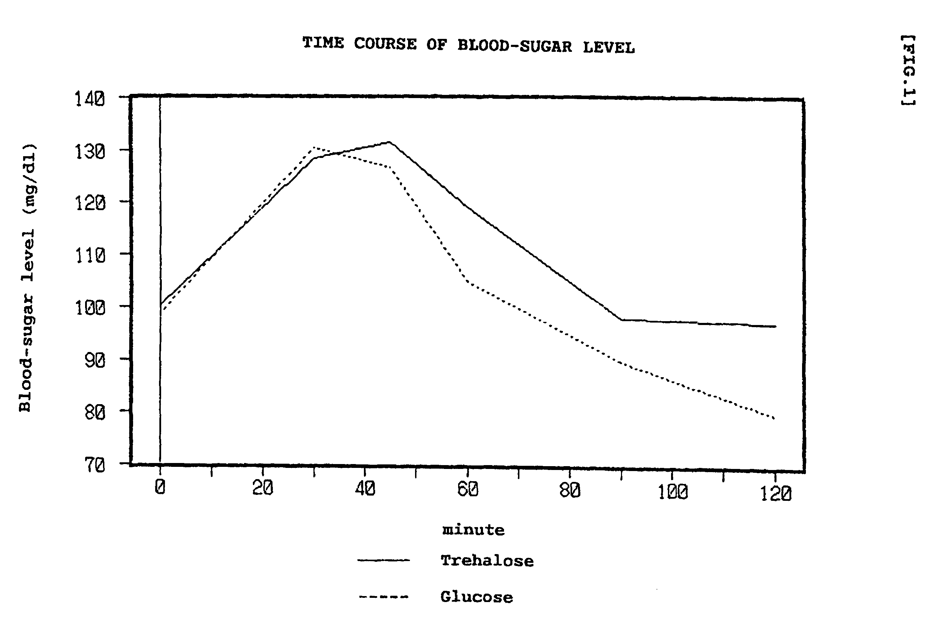

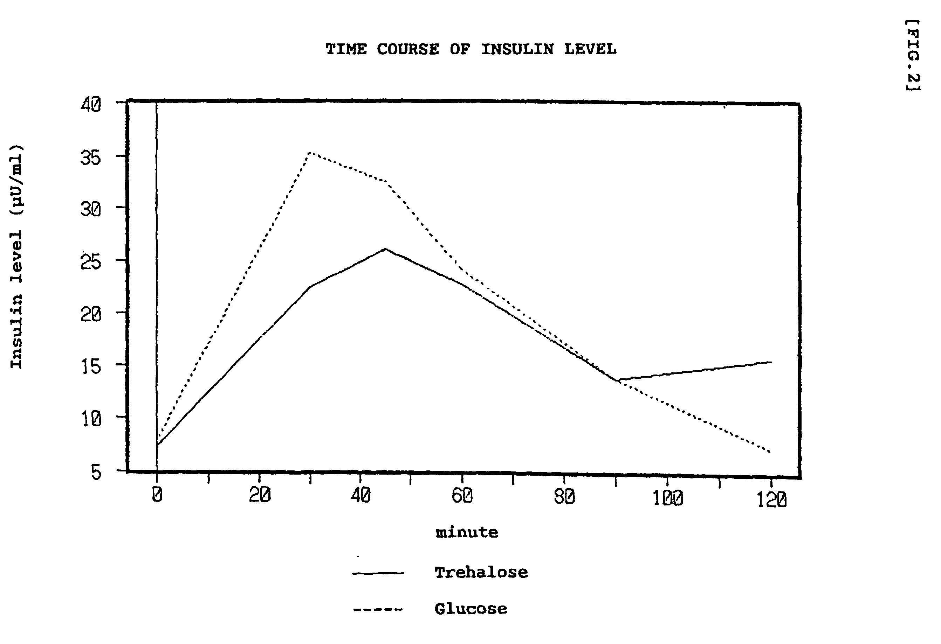

In accordance with the method reported by H. Atsuji et al. in *Journal of Clinical Nutrition,* Vol.41, No.2, pp. 200-208 (1972), 30g of a hydrous crystalline trehalose prepared by the method in Experiment 1-2 was dissolved in water into a 20 w/v % aqueous trehalose solution, and prescribed amounts of which were orally administered to 6 male volunteers, 26-31-year-old. The volunteers were let blood at a prescribed time interval, and each blood sample was measured its blood sugar level, i.e. glucose concentration (mg/dl) and insulin level ($\mu$U/ml). As a control, glucose was used. The results of both levels were the mean values of 6 volunteers. FIGs.1 and 2 show the time courses of blood sugar- and insulin-levels, respectively. In the figures, the solid- and dashed-lines mean the time courses of trehalose and insulin, respectively.

As evident from the results in FIGs.1 and 2, although the dynamics of the volunteers administered with trehalose tended to show a time lag against those of the volunteers with glucose the volunteers with trehalose showed the similar dynamics as those with glucose, namely trehalose induced the maxima of blood sugar- and insulin-levels at about 30-45 min after the administration. Unlike the results of digestion test *in vivo* in Experiment 2, the results in this Experiment revealed that trehalose is readily assimilated, metabolized and utilized by living bodies as an energy source when orally administered.

Experiment 4

Utilization test *in vivo* by parenteral administration

Experiment 4-1

Rapid administration

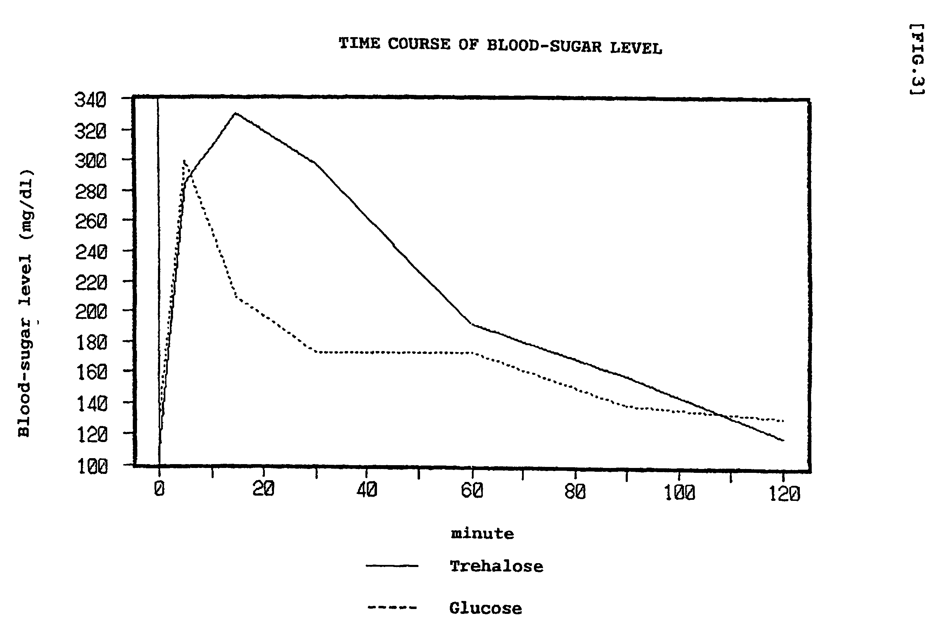

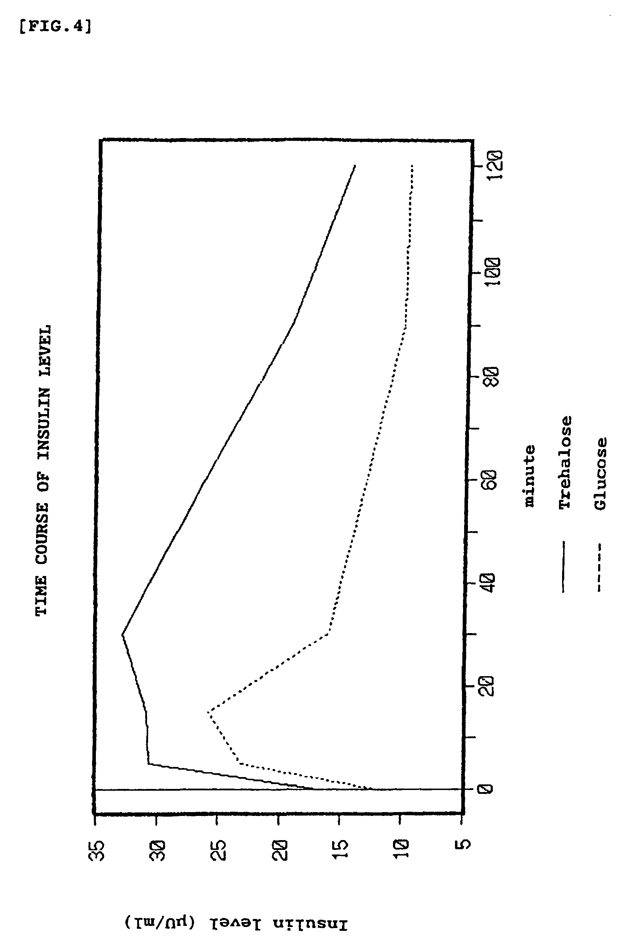

A hydrous crystalline trehalose prepared by the method in Experiment 1-2 was dissolved in a refined water, and the resultant solution was membrane filtered, concentrated and recrystallized to obtain a pyrogen-free hydrous crystalline trehalose. The crystal thus obtained was dissolved in injectable distilled water into a 10 w/v % solution isotonic to rabbit blood as a trehalose infusion. By using 6 rabbits, about 2-3kg weight, the infusion was rapidly administered to their ear veins at a dose of one g/kg body weight within 1.5 min, followed by collecting their blood at a prescribed time interval, and subjecting the collected blood samples to the measurements of their sugar levels (glucose concentration (mg/dl)) and insulin levels ($\mu$U/ml). As a control, 5 w/v % glucose solution isotonic to rabbit blood was similarly administered to rabbit veins at a dose of 0.5g/kg body weight. Each result was expressed by the mean value of the 6 rabbits, and the time courses of the blood sugar- and insulin-levels of the rabbits were respectively shown in FIGs.3 and 4. In the figures, the solid- and dashed-lines mean the time courses of trehalose and insulin in rabbit blood, respectively.

As evident from the results in FIGs.3 and 4, both the dynamics of blood sugar- and insulin-levels of the rabbits administered with trehalose tended to show a time lag against those of the rabbits with glucose, namely the maxima were found about 5-30 min after their administrations. These results revealed that when trehalose is parenterally administered rapidly it is readily hydrolyzed into glucose *in vivo*, metabolized and utilized by living bodies as an energy source. The amounts of saccharides in urine secreted by the rabbits were monitored from the initiation of the administrations to 6 hours after the administrations, and the results revealed that the amount of trehalose secreted in rabbit urine was absolutely low similarly as in the case of glucose, i.e. the amount was lower than 10% of the administered trehalose, d.s.b.

Experiment 4-2

Slow administration

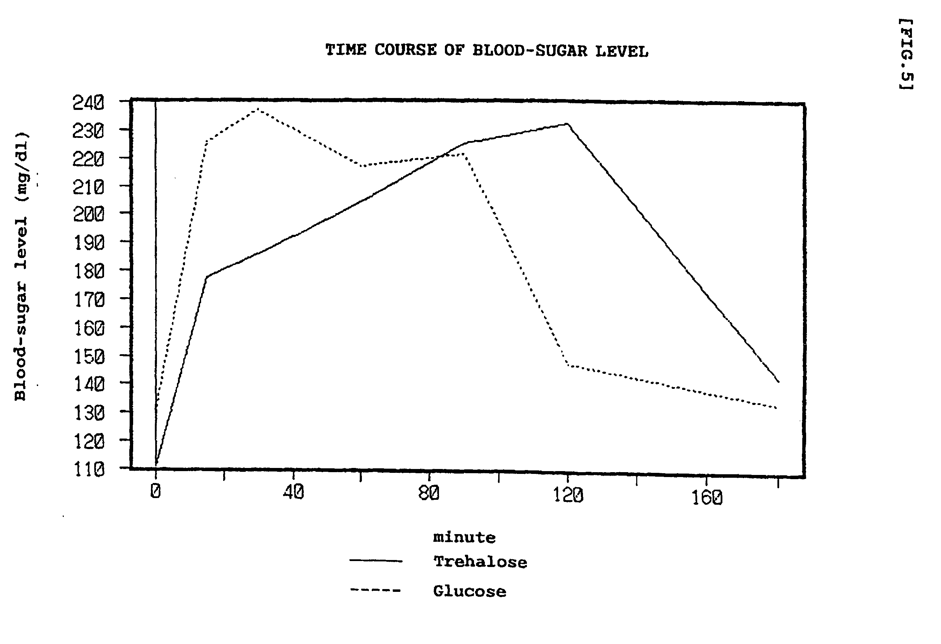

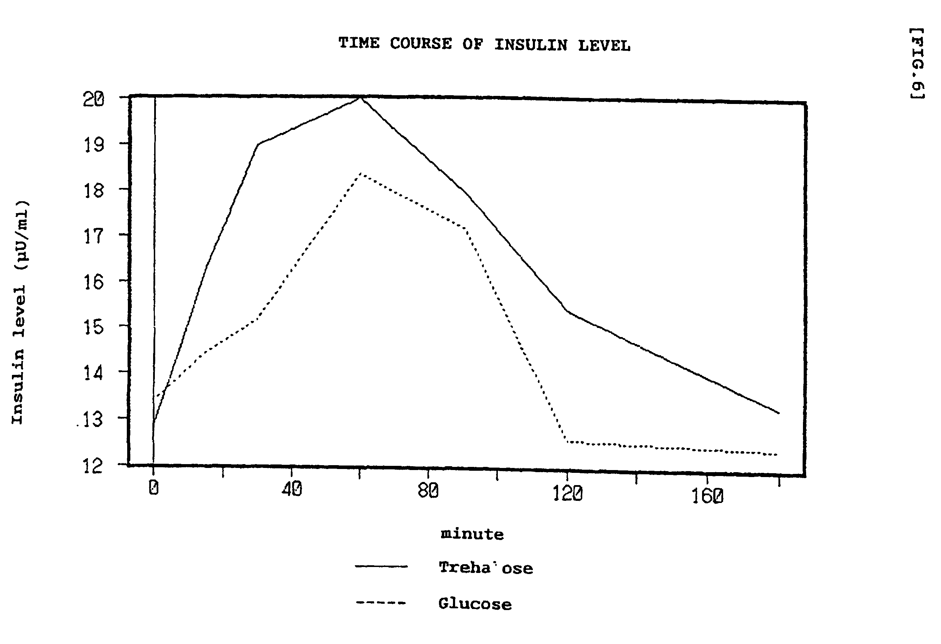

A trehalose infusion prepared by the method in Experiment 4-1 was administered to 6 rabbits, about 2-3kg weight, and collected their blood at a prescribed time interval, and the collected blood samples were measured their blood-sugar levels (glucose concentration (mg/dl)) and insulin levels ($\mu$U/ml) similarly as the method in Experiment 4-1 except that the infusion was slowly administered to the rabbit ear veins over a period of 1.5 hours. Each result was expressed by the mean value of the 6 rabbits, and the time courses of the blood sugar- and insulin-levels of the rabbits were respectively shown in FIGs.5 and 6.

As evident from the results in FIGs.5 and 6, although the dynamics of blood-sugar level of the rabbits administered with trehalose tended to show a time lag against those of the rabbits with glucose, both the dynamics were substantially the same, and the dynamics of insulin level of the rabbits with trehalose were substantially the same as those with glucose. These results revealed that when trehalose is parenterally administered slowly to living bodies, it is readily hydrolyzed into glucose *in vivo*, metabolized and utilized by the living bodies as an energy source. The amounts of saccharides secreted in the rabbit urine were monitored from the initiation of the administrations to 6 hours after the administrations, and the results revealed that the amount of trehalose secreted in each urine was absolutely low similarly as in the case of glucose, i.e. the amount was lower than 10% of the administered trehalose, d.s.b.

Experiment 5

Acute toxicity test

By using mice, trehalose was tested for its acute toxicity by orally administering it to the mice a hydrous crystalline trehalose prepared by the method in Experiment 1-2. As a result, it was revealed that trehalose is a substance having a relatively-low toxicity, and no mouse died even when administered with the highest possible dose. Based on the result, the $LD_{50}$ was 50g/kg or higher, though the value was not so accurate.

The following examples relate to energy-supplementing compositions containing trehalose as an effective ingredient; the examples showing a use according to present claim 1 are embodiments of the present invention. The other examples are background examples which are not part of the present invention.

Example 1

Chocolate

Forty parts by weight of cacao paste, 10 parts by weight of cacao butter, and 50 parts by weight of a hydrous crystalline trehalose prepared by the method in Experiment 1-2 were mixed, and the resultant mixture was passed through a refiner to reduce the particle size, transferred to a conche, and kneaded at 50°C for 2 days. During this kneading step, 0.5 parts by weight of lecithin was added to the contents, and the resultant mixture was sufficiently kneaded. Thereafter, the mixture was kept at 31°C by a thermoregulator, poured into a mold before the solidification of cacao butter, deaerated with a vibrator, and solidified while passing through a cooling tunnel at 10°C for 20 min. The solidified contents were removed from the mold and packaged to obtain the desired product.

The product substantially free of hygroscopicity has a satisfiable color, gloss and texture, and mildly melts in the mouth to exert a high-quality sweetness and flavor, and these render it arbitrarily useful as an energy-supplementing composition.

Example 2

Chewing gum

Three parts by weight of a gum base was melted by heating until it softened, mixed with 4 parts by weight of sucrose and 3 parts by weight of a crystalline trehalose powder prepared by the method in Experiment 1-3, and further mixed with adequate amounts of a flavor and a coloring agent. The resultant mixture was in usual manner kneaded by a roll, formed and packaged to obtain the desired product.

The product, a chewing gum with a satisfiable texture and flavor, can be arbitrarily used as an energy-supplementing composition.

Example 3

Hard candy

One hundred parts by weight of a 55% sucrose solution was mixed while heating with 30 parts by weight of a high trehalose content solution prepared by the method in Experiment 1-4, and the resultant mixture was concentrated *in vacuo* under heating conditions up to give a moisture content of lower than 2%. The concentrate was admixed with one part by weight of citric acid and adequate amounts of a lemon flavor and a coloring agent, and the mixture was formed in usual manner to obtain the desired product.

The product, wherein the crystallization of sucrose is well inhibited, is a high-quality hard candy having a satisfiable taste and biting property, and suitably used as an energy-supplementing composition.

Example 4

Custard cream

One hundred parts by weight of corn starch, 100 parts by weight of a trehalose syrup containing 70% of a crystalline trehalose powder, d.s.b., prepared by the method in Experiment 1-3, 80 parts by weight of maltose, 20 parts by weight of sucrose, and one part by weight of salt were sufficiently kneaded, and the mixture was further admixed with 280 parts by weight of fresh egg while stirring, and gradually mixed with 1,000 parts by weight of a boiling milk. The resultant mixture was continued stirring while heating, and the heating was suspended when the whole contents became semitransparent, followed by cooling the resultant and adding thereto an adequate amount of a vanilla flavor. The product was weighed, injected into a container and packaged to obtain the desired product.

The product has a smooth surface and a mild sweetness and taste, and these render it arbitrarily useful as an energy-supplementing composition.

Example 5

*Uiro-no-moto* (premix of *uiro*)

A *uiro-no-moto* was prepared by sufficiently mixing 90 parts by weight of rice powder with 20 parts by weight of corn starch, 120 parts by weight of a crystalline trehalose powder prepared by the method in Experiment 1-3, and 4 parts by weight of pullulan. The product was kneaded with adequate amounts of matcha (powdered green tea) and water, and the mixture was placed in a container and steamed up for 60 min to obtain a *matcha-uiro*.

The product has a satisfiable gloss, biting property and flavor. The retrogradation of starch in the product is well inhibited, and this attains a satisfiable shelf-life. The product can be arbitrarily used as an energy-supplementing composition.

Example 6

Lactic acid beverage

Ten parts by weight of defatted milk was sterilized by heating at 80°C for 20 min, cooled to 40°C, mixed with 0.3 parts by weight of a starter, and fermented at about 37°C for 10 hours. Thereafter, the mixture was homogenized and mixed with 4 parts by weight of a crystalline trehalose powder prepared by the method in Experiment 1-3, one part by weight of sucrose, and 2 parts by weight of isomerized syrup, and the resultant mixture was sterilized by heating at 70°C. The product thus obtained was cooled, mixed with an adequate amount of a flavor, and bottled to obtain the desired product.

The product, a high-quality lactic acid beverage with a well harmonized flavor and sweetness, can be arbitrarily used as an energy-supplementing composition.

Example 7

Powdered juice

Thirty-three parts by weight of a powdered orange juice prepared by spray-drying was mixed to homogeneity while stirring with 50 parts by weight of a hydrous crystalline trehalose powder prepared by the method in Experiment 1-2, 10 parts by weight of sucrose, 0.65 parts by weight of anhydrous citric acid, 0.1 part by weight of malate, 0.1 part by weight of L-ascorbic acid, 0.1 part by weight of sodium citrate, 0.5 parts by weight of pullulan, and an adequate amount of a powdered flavor. The mixture was pulverized, fed to a fluidized-bed granulator wherein the exhaust temperature and flow rate were respectively adjusted to 40°C and 150m$^3$/min, sprayed with a high trehalose content solution, as a binder, prepared with a commercially available trehalose, and granulated for 30 min. The resultant granules in powder were weighed and packaged to obtain the desired product.

The product, a 30% powdered juice, d.s.b., is stable for a relatively-long period of time without giving an unfavorable taste and smell and causing solidification by absorbing moisture. Thus, the product can be arbitrarily used as an energy-supplementing composition.

Example 8

Powdered egg yolk

Egg yolks prepared from fresh eggs were sterilized by a plate heater at 60-64°C, and one part by weight of the resultant liquid was mixed with 4 parts by weight of an anhydrous crystalline trehalose prepared by the method in Experiment 1-4. The resultant mixture was placed in a container and allowed to stand overnight to form a block while allowing trehalose to convert into hydrous crystalline trehalose. The block was pulverized by a cutter to obtain a powdered egg yolk.

The product can be arbitrarily used as a material for confectioneries such as a premix, ice cream and candy, and emulsifier, as well as an energy-supplementing composition for oral and intubation feedings.

Example 9

Solid preparation for intubation feeding

A composition consisting of the following compositions was prepared: Five hundred parts by weight of hydrous crystalline trehalose prepared by the method in Experiment 1-2, 270 parts by weight of powdered egg yolk, 209 parts by weight of defatted milk, 4.4 parts by weight of sodium chloride, 1.8 parts by weight of potassium chloride, 4 parts by weight of magnesium sulfate, 0.01 part by weight of thiamine, 0.1 part by weight of sodium ascorbate, 0.6 parts by weight of vitamin E acetate, and 0.04 parts by weight of nicotinamide. Twenty-five g aliquots of the composition were injected into moisture-proof laminated small bags and heat sealed to obtain the desired product.

One bag of the product is dissolved in about 150-300ml of water into a fluid food, and orally administered or parenterally administered to nasal cavity, stomach and intestines by intubation feeding as an energy-supplementing composition.

Example 10

Hyperalimentation

A hydrous crystalline trehalose powder, prepared by the method in Experiment 1-2, was dissolved in water into an about 10 w/v % aqueous trehalose solution which was then in usual manner membrane filtered to remove pyrogen, aseptically injected into a plastic bottle, and sealed to obtain the desired product.

The product, a satisfactorily stable hyperalimentation substantially free of change on standing, is suitable for intravenous- and intraperitoneal-administrations. A 10 w/v % solution of the product is isotonic to blood, and this means it supplements energy to living bodies at 2-fold higher concentration than in the case of glucose.

Example 11

Hyperalimentation

A hydrous crystalline trehalose, prepared by the method in Experiment 1-2, and an amino acid composition consisting of the following components were dissolved by stirring in water to give 5 w/v % and 30 w/v %, respectively, and, similarly as in Example 10 the resultant solution was purified into a pyrogen-free solution which was then injected into a plastic bottle and sealed to obtain the desired product.

| Components of amino acid composition | |
| --- | --- |
| Component | mg/100 ml |
| L-Isoleucine | 180 |
| L-Leucine | 410 |
| L-Lysine monohydrochloride | 620 |
| L-Methionine | 240 |
| L-Phenyl alanine | 290 |
| L-Threonine | 180 |
| L-Tryptophane | 60 |
| L-Valine | 200 |
| L-Arginine hydrochloride | 270 |
| L-Histidine monohydrochloride | 130 |
| Glycine | 340 |

Although the product is a multiple hyperalimentation of trehalose and amino acids, it is satisfactorily stable without substantial change on standing and can be suitably administered intravenously and intraperitoneally to living bodies. The product can be favorably used as an energy-supplementing composition to supplement energy as well as amino acids.

Example 12

Traumatic ointment

Five hundred parts by weight of a crystalline trehalose powder, prepared by the method in Experiment 1-3, was admixed with 50 parts by weight of methanol solution containing 3 parts by weight of iodine, and the resultant solution was mixed with 200 parts by weight of a 10 w/v % aqueous pullulan solution to obtain a traumatic ointment having a satisfiable extensibility and adhesiveness.
The iodine contained in the product exerts a bactericidal activity, and the trehalose in the product acts as an energy-supplementing saccharide source on viable cells, and because of these the product shortens a healing period and satisfactorily heals a wound surface.

Example 13

Sugar coated tablet

A crude tablet, 150mg weight, as a core, was coated until it weighed about 230mg, d.s.b., with a solution consisting of 40 parts by weight of a hydrous crystalline trehalose prepared by the method in Experiment 1-2, 2 parts by weight of pullulan having an average molecular weight of 200,000, 30 parts by weight of water, 25 parts by weight of talc, and 3 parts by weight of titanium oxide. The resultant tablet was further coated with 65 parts by weight of a fresh preparation of the same hydrous crystalline trehalose, one part by weight of pullulan, and 34 parts by weight of water. The tablet thus obtained was glossed with a wax solution to obtain the desired product with a satisfiable appearance.
The product, which is readily preparable by the above-mentioned coating step, has a relatively-high shock-tolerance, and retains its high quality for a relatively-long period of time. Thus, it can be suitably used as an energy-supplementing composition.

As evident from above, the present energy-supplementing saccharide source essentially consisting of trehalose, prepared by the method containing a step of allowing a non-reducing saccharide-forming enzyme to act on a reducing partial starch hydrolysate, has a satisfiable stability without substantially exhibiting a reducing power, as well as possessing a character of being readily metabolized *in vivo* and utilized by living bodies as an energy source. Furthermore, the present energy-supplementing composition, containing as an effective ingredient trehalose prepared from reducing partial starch hydrolysates by a novel biochemical technique, has a character that it can be readily prepared into more satisfiable multiple nutritive compositions in combination with other nutritives and medicaments, as well as into pharmaceutical compositions with an increased therapeutic efficacy.

## Claims

1. Use of $\alpha,\alpha$-trehalose to prepare a composition to be administered parenterally to living bodies for supplying energy to a living body,
   wherein the $\alpha,\alpha$-trehalose is preparable by the method comprising a step of allowing a non-reducing saccharide-forming enzyme to act on a partial starch hydrolysate, which exhibits a reducing power, to form a saccharide solution comprising trehalose; and optionally feeding said saccharide solution to column chromatography using a strong acidic cation exchange resin to increase the purity of the trehalose.

2. Use according to claim 1, wherein the composition contains at least 10 w/w % trehalose on a dry solid basis.

3. Use according to claim 1 or 2, wherein the composition comprises trehalose and another saccharide(s).

## Patentansprüche

1. Verwendung von $\alpha,\alpha$-Trehalose zur Herstellung einer Zusammensetzung, die zur Energiezuführung an einen lebenden Organismus parenteral an lebende Organismen zu verabreichen ist, wobei die $\alpha,\alpha$-Trehalose herstellbar ist durch das Verfahren, umfassend einen Schritt des Zulassens, dass ein nicht reduzierendes, Saccharid-bildendes Enzym auf ein Stärke-Teilhydrolysat, das ein Reduktionsvermögen aufweist, einwirkt, um eine Trehalose umfassende Saccharidlösung zu bilden; und gegebenenfalls des Zuführens der Saccharidlösung einer Säulenchromatographie unter Verwendung eines stark sauren Kationenaustauscherharzes, um die Reinheit der Trehalose zu steigern.

2. Verwendung gemäß Anspruch 1, wobei die Zusammensetzung zumindest 10 Gew.% Trehalose auf Basis eines trockenen Feststoffs enthält.

3. Verwendung gemäß Anspruch 1 oder 2, wobei die Zusammensetzung Trehalose und ein weiteres Saccharid/weitere Saccharide umfasst.

## Revendications

1. Utilisation d'$\alpha,\alpha$-tréhalose pour la préparation d'une composition destinée à être administrée par voie parentérale à des organismes vivants pour fournir de l'énergie à un organisme vivant, dans laquelle l'$\alpha,\alpha$-tréhalose peut être préparée par le procédé comprenant une étape consistant à laisser une enzyme de formation de saccharides non réducteurs agir sur un hydrolysat partiel d'amidon, qui présente un pouvoir réducteur, pour former une solution de saccharides comprenant du tréhalose ; et, facultativement, à soumettre ladite solution de saccharides à une chromatographie sur colonne en utilisant une résine échangeuse de cations du type acide fort pour accroître la pureté du tréhalose.

2. Utilisation suivant la revendication 1, dans laquelle la composition contient au moins 10 % en poids/poids de tréhalose sur la base des matières solides sèches.

3. Utilisation suivant la revendication 1 ou 2, dans laquelle la composition comprend du tréhalose et un ou plusieurs autres saccharides.

[FIG.1]

EP 0 619 951 B1

TIME COURSE OF BLOOD-SUGAR LEVEL

12

[FIG.2]

TIME COURSE OF INSULIN LEVEL

minute

Trehalose ——
Glucose ------

Insulin level (µU/ml)

[FIG.3]

TIME COURSE OF BLOOD-SUGAR LEVEL

Blood-sugar level (mg/dl)

minute

Trehalose ————

Glucose ------

TIME COURSE OF INSULIN LEVEL

[FIG.4]

EP 0 619 951 B1

15

[FIG.5]

TIME COURSE OF BLOOD-SUGAR LEVEL

Blood-sugar level (mg/dl)

minute

—————— Trehalose

----- Glucose

EP 0 619 951 B1

16

[FIG.6]

TIME COURSE OF INSULIN LEVEL